Europäisches Patentamt

(19) European Patent Office

Office européen des brevets

(11) Numéro de publication: **0 290 327**
**A1**

# DEMANDE DE BREVET EUROPEEN

(12)

(21) Numéro de dépôt: **88401052.1**

(22) Date de dépôt: **29.04.88**

(51) Int. Cl.⁴: **G 06 F 15/02**
**A 45 D 44/00**

(30) Priorité: **30.04.87 FR 8706204**

(43) Date de publication de la demande:
**09.11.88 Bulletin 88/45**

(84) Etats contractants désignés:
**AT BE CH DE ES FR GB GR IT LI LU NL SE**

(71) Demandeur: **SCHWARZKOPF FRANCE**
**Zone Industrielle rue de la Demi-Lune B.P. 385**
**F-86010 Poitiers Cédex (FR)**

**STE 3 C I**
**79, avenue Aristide Briand**
**F-93190 Livry Gargan (FR)**

(72) Inventeur: **Marchand, Jean-Claude**
**7, rue Victor Hugo**
**F-92300 Levallois Perret (FR)**

**Guerin, Christian**
**7, Allée des Acacias**
**F-92310 Sevres (FR)**

**Fourneau, Didier**
**3, rue de la Liberté**
**F-94500 Champigny (FR)**

(74) Mandataire: **Joly, Jean-Jacques et al**
**CABINET BEAU DE LOMENIE 55, rue d'Amsterdam**
**F-75008 Paris (FR)**

(54) **Appareil pour déterminer automatiquement la formule de coloration de cheveux afin d'obtenir une coloration souhaitée.**

(57) Des moyens de saisie de données tels qu'un clavier (20) sont prévus pour fournir à une unité de traitement à microprocesseur une information représentative de la coloration souhaitée et des informations d'état chacune représentative de la valeur d'un paramètre parmi un ensemble de paramètres prédéterminés, caractérisant les cheveux à colorer. L'unité de traitement comprend des moyens pour sélectionner la formule de coloration convenable en fonction des données saisies et à partir de formules stockées en mémoire, et pour commander l'affichage de la formule sur un dispositif d'affichage (30)

Fig. 2

## Description

## APPAREIL POUR DETERMINER AUTOMATIQUEMENT LA FORMULE DE COLORATION DE CHEVEUX AFIN D'OBTENIR UNE COLORATION SOUHAITEE

La présente invention concerne un appareil pour déterminer automatiquement la formule de coloration de cheveux afin d'obtenir une coloration souhaitée.

Il est parfois difficile, même pour un spécialiste expérimenté d'obtenir exactement et parfaitement une coloration et un reflet désirés sur des cheveux quelles que soient les caractéristiques des cheveux à colorer.

Une assistance donnant la formule de coloration à mettre en oeuvre au moyen de produits d'une gamme déterminée, pour arriver le mieux possible au résultat souhaité compte tenu des caractéristiques particulières des cheveux à colorer, est donc souhaitable.

L'invention a précisément pour but de fournir un appareil permettant de réaliser cette fonction d'assistance de façon automatique et fiable tout en présentant une très grande facilité d'emploi.

Ce but est atteint au moyen d'un appareil comportant, conformément à l'invention :
- une unité de traitement à microprocesseur,
- au moins une mémoire contenant des formules de coloration,
- des moyens de saisie de données pour fournir à l'unité de traitement une information représentative de la coloration souhaitée et des informations d'état chacune représentative de la valeur d'un paramètre parmi un ensemble de paramètres prédéterminés, caractérisant les cheveux à colorer, et
- un dispositif d'affichage,
- l'unité de traitement comprenant des moyens pour sélectionner une formule de coloration en fonction des données saisies et pour commander l'affichage de la formule sélectionnée sur le dispositif d'affichage.

Les paramètres caractérisant les cheveux à colorer comprennent, notamment, l'état des cheveux (c'est-à-dire si les cheveux sont naturels, colorés avec ou sans oxydation ou décolorés), la hauteur de ton des cheveux, le reflet des longueurs, la longueur des racines, le pourcentage de cheveux blancs et la grosseur des cheveux. D'autres paramètres peuvent être pris en compte pour élaborer une formulation plus fine, comme par exemple la qualité des pointes, l'existence ou non de mèches,...

De préférence, des moyens d'interrogation sont prévus pour demander la saisie des informations d'état représentatives des paramètres dans un ordre déterminé, ceci afin d'optimiser le processus de sélection de la formule de coloration.

Suivant un mode de réalisation de l'appareil conforme à l'invention, les moyens de saisie de données comportent un clavier comprenant des touches dont chacune correspond à une valeur particulière d'un paramètre. Les touches sont avantageusement groupées en plusieurs ensembles correspondant chacun à un paramètre. Des moyens indicateurs, par exemple lumineux, sont associés aux ensembles de touches pour indiquer le paramètre dont la valeur est à saisir afin que les informations d'état soient saisies dans l'ordre convenable. Avantageusement encore, les touches constituent également des touches d'un clavier alphanumérique utilisé par exemple pour saisir l'information représentative de la teinte souhaitée et, le cas échéant, d'autres informations, par exemple relatives à l'identité de la personne dont les cheveux sont à colorer.

D'autres modes de réalisation des moyens de saisie de données pourront être utilisées comme, par exemple, des moyens de sélection (touches ou souris) d'informations affichées sur un écran sous la commande de l'unité de traitement.

Selon encore une autre particularité avantageuse de l'appareil conforme à l'invention, l'unité de traitement, la mémoire, les moyens de saisie de données et le dispositif d'affichage sont regroupés dans un même boîtier. Sur le boîtier pourra être en outre disposée une palette montrant les différentes valeurs pouvant être saisies pour la hauteur de ton de cheveux constituant l'un des paramètres des cheveux à colorer.

La formule de coloration sélectionnée est affichée par exemple par imprimante qui fournit un ticket imprimé.

Ainsi l'appareil, conforme à l'invention, permet sous une forme compacte et facilement transportable de disposer d'un outil fiable et simple à manier donnant rapidement la formule de coloration la mieux adaptée au problème posé.

L'invention sera mieux comprise à la lecture de la description donnée ci-après en référence aux dessins annexés sur lesquels :
- la figure 1 est un schéma par blocs illustrant de façon très générale la structure d'un appareil conforme à l'invention ;
- la figure 2 est une vue générale d'un mode particulier de réalisation d'un appareil conforme à l'invention, montrant notamment le clavier de l'appareil ; et
- la figure 3 est un organigramme illustrant des étapes successives d'un exemple de procédure de recherche de formule de coloration au moyen de l'appareil conforme à l'invention.

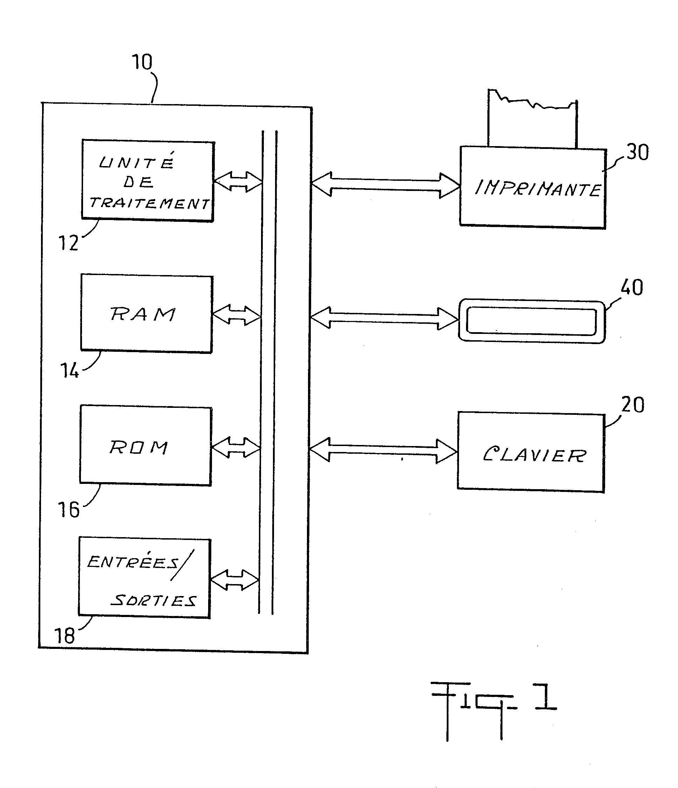

L'appareil illustré très schématiquement par la figure 1 comprend un circuit 10 à microprocesseur auquel sont reliés un clavier 20, une imprimante 30 et un afficheur à cristaux liquides 40.

De façon classique, le circuit 10 à microprocesseur comprend notamment une unité de traitement 12, des circuits de mémoire vive (RAM) 14, des circuits de mémoire morte (ROM) 16 et des circuits d'entrée/sortie. Dans les circuits de mémoire morte 16 sont stockés les programmes nécessaires au fonctionnement de l'appareil ainsi que des informations fixes relatives, d'une part, à l'ensemble des produits constituant une gamme de produits parmi lesquels sont choisis ceux utilisés pour mettre en oeuvre les formules de coloration et, d'autre part, à un ensemble de formules de coloration ou, plus exactement, à un ensemble de types de formules de coloration.

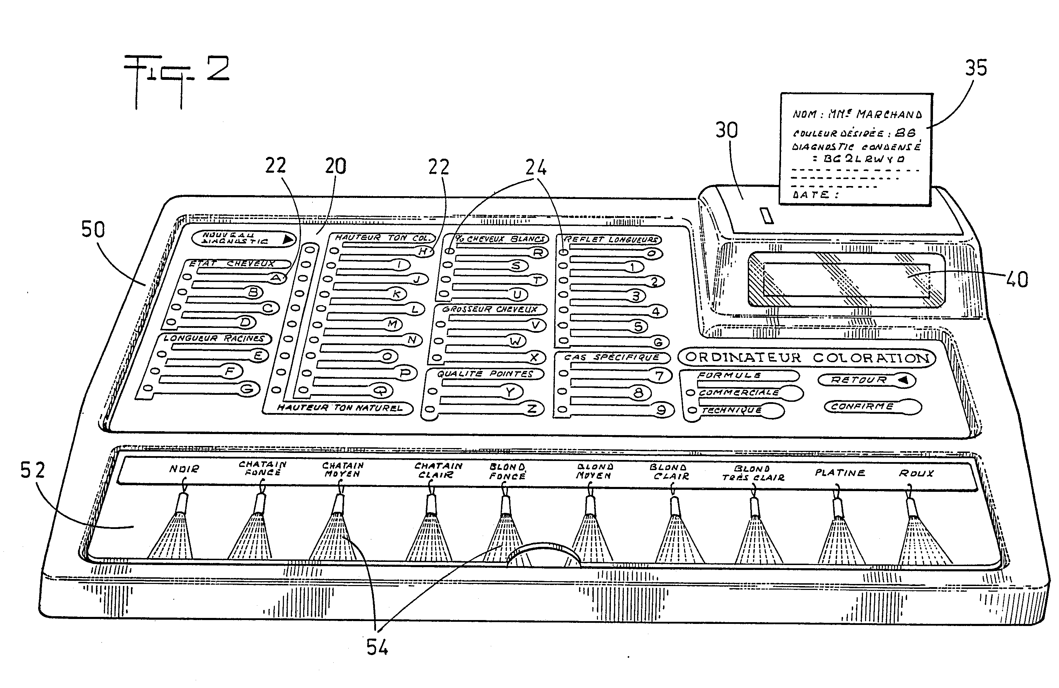

Une forme de réalisation d'un appareil selon l'invention est montrée de façon plus détaillée sur la figure 2.

Le circuit 10, le clavier 20, l'imprimante 30 et l'afficheur 40 sont réunis dans un boîtier 50.

Le clavier 20 occupe la moyenne partie de la face supérieure du boîtier 50. Le clavier comporte des touches 22, par exemple du type à sensation tactile, destinées à saisir des informations relatives, d'une part, à la coloration souhaitée et, d'autre part, aux valeurs d'un certain nombre de paramètres caractérisant les cheveux à colorer. D'autres informations peuvent être saisies comme, par exemple, le nom de la personne dont les cheveux sont à colorer. Dans l'exemple illustré, la plupart des touches ont deux fonctions ; selon l'étape de la procédure en cours, elles constituent soit des touches d'un clavier alphanumérique, soit des touches correspondant aux différentes valeurs attribuables aux différents paramètres utilisés pour caractériser des cheveux à colorer.

Les touches sont réparties en plusieurs ensembles chacun correspondant à un paramètre particulier. Ainsi, on trouve :

- un premier ensemble de quatre touches A à D permettant de caractériser l'état des cheveux : cheveux jamais colorés (A), colorés par oxydation (B), colorés sans oxydation (C), décolorés ou éclaircis de plus de quatre tons (D),

- un deuxième ensemble de trois touches E à G permettant de caractériser la longueur des racines : plus de 1 an (E), 1 an et moins (F), pas de racines (G),

- un troisième ensemble de dix touches H à Q permettant de caractériser la hauteur de ton des cheveux (soit la hauteur de ton naturel si les cheveux à colorer sont naturels, soit la hauteur de tons des cheveux colorés ou décolorés si les cheveux à colorer sont déjà colorés ou décolorés) : noir (H), châtain foncé (I), châtain moyen (J), châtain clair (K), blond foncé (L), blond moyen (M), blond clair (N), blond très clair (0), blanc ou platine (P), roux (Q),

- un quatrième ensemble de quatre touches R à U permettant de caractériser le pourcentage de cheveux blancs : moins de 30% (R), de 30 à 50% (S), de 50 à 80% (T), plus de 80% (U),

- un cinquième ensemble de trois touches V à X permettant de caractériser la grosseur des cheveux : gros (V), moyens (W), fins (X),

- un sixième ensemble de deux touches Y, Z permettant de caractériser la qualité des pointes : saines et uniformes (Y), poreuses et délavées (Z),

- un septième ensemble de sept touches 0 à 6 permettant de caractériser le reflet des longueurs : naturel ou cendré (0), doré (1), marron ou châtaigne (2), acajou ou violine (3), cuivré rouge (4), cuivré doré (5), mat ou verdâtre (6), et

- un huitième ensemble de trois touches 7 à 9 permettant de caractériser un cas spécifique : pas de mèches (7), mèches à conserver (8), mèches à supprimer (9).

On note encore un neuvième ensemble de touches permettant de choisir, le cas échéant, entre une formule dite "commerciale" et une formule dite "technique", la seconde devant donner un résultat de qualité supérieure mais à un coût de produits plus élevé et/ou une durée de traitement plus longue.

Deux touches "retour à la question précédente" et "confirme" sont utilisables lors de la procédure d'interrogation au cours de laquelle les différentes valeurs des paramètres des cheveux à colorer sont saisies par action sur les touches.

Enfin, une touche "nouveau diagnostic" permet de mettre en marche l'appareil et démarrer la procédure de recherche de formule de coloration.

De préférence, les différentes valeurs de paramètres doivent être saisies dans un ordre particulier afin d'optimiser la procédure de recherche. Cet ordre est conforme à celui dans lequel les différents ensembles de touches sont décrits ci-avant. A l'effet de respecter l'ordre de saisie de paramètres, des moyens indicateurs sont prévus pour indiquer à l'utilisateur le paramètre à évaluer à chaque stade de la procédure. Dans l'exemple illustré, les moyens indicateurs consistent dans des diodes électroluminescentes, telles que 24, associées aux touches correspondant aux valeurs de paramètre. Lorsque la valeur d'un paramètre doit être saisie, les diodes associées à l'ensemble de touches correspondant sont allumées ; le choix d'une valeur par action sur la touche correspondante peut alors commander l'extinction de la diode associée à cette touche, ce qui permet à l'utilisateur de visualiser son choix avant de le confirmer. On notera que deux ensembles de diodes sont associées aux touches relatives à la hauteur de ton des cheveux : un premier ensemble étant activé lorsque les cheveux ont été préalablement identifiés comme naturels et le deuxième ensemble étant activé lorsque les cheveux ont été préalablement identifiés comme colorés ou décolorés.

L'allumage des diodes 24 est commandé par le circuit à microprocesseur.

A la face supérieure du boîtier 50, on voit encore l'écran de l'afficheur 40 et la sortie papier de l'imprimante 30.

L'afficheur à cristaux liquides 40 comprend par exemple un écran de deux lignes de 20 caractères permettant d'afficher des informations alphanumériques entrées au moyen du clavier ou des messages élaborés par l'unité de traitement adressés à l'utilisateur au cours de la procédure de recherche de formule de coloration.

L'imprimante 30 est par exemple de type thermique pour éditer la formule de coloration déterminée sous une forme imprimée sur un support papier, par exemple en forme de ticket 32 découpé d'un rouleau de stockage. Eventuellement, le ticket porte d'autres informations comme le nom de la personne dont les cheveux sont à colorer et la date.

On note enfin le long du bord avant du boîtier, une palette de tons 52 sous forme de mèches teintes 54 dans les différentes valeurs attribuables au ton des cheveux à colorer, afin d'aider à l'évaluation de ce paramètre.

Bien entendu, il est également prévu une palette (non représentée) donnant les différentes colorations réalisables avec tous les produits de la gamme de produits considérée. Comme déjà indiqué, des informations caractérisant tous ces différents produits de coloration sont mémorisées dans les circuits de mémoire de l'appareil. Pour chaque produit, sont mémorisés sa référence dans la gamme et un code numérique déterminé, par exemple, en utilisant :

    - un premier chiffre indiquant la hauteur de ton de la coloration obtenue par le produit,

    - un deuxième chiffre éventuel indiquant le reflet principal,

    - un troisième chiffre éventuel indiquant le reflet secondaire,

    - un quatrième chiffre éventuel indiquant l'éclat.

A chaque produit, on pourra également associer les références des produits qui s'en distinguent par un écart d'un ton plus foncé et d'un ton plus clair, ceci afin de déterminer facilement la formule de coloration convenable lorsque celle-ci impose, en raison de caractéristiques particulières des cheveux et/ou de la coloration à obtenir, l'utilisation du produit un ton plus clair ou un ton plus foncé que celui correspondant normalement à la coloration souhaitée.

Les circuits de mémoire contiennent également un ensemble de formules de coloration dont la solution est effectuée par l'unité de traitement en fonction de la coloration souhaitée et des paramètres saisis. Il pourra s'agir en fait de formules types de coloration dans lequelles la référence du produit de coloration n'est pas spécifiée, la formule à utiliser étant obtenue en sélectionnant la formule type puis en complétant celle-ci par la référence de produit correspondant à la coloration souhaitée (ou, dans certains cas du produit un ton plus clair ou un ton plus foncé, comme évoqué plus haut).

Le nombre de formules types de coloration peut être très élevé : plusieurs centaines pour couvrir les différents cas possibles lorsqu'un choix est offert entre plusieurs dizaines de colorations différentes. Pour cette raison, on se bornera ici à donner quelques exemples de modes de sélection de formules types. Il va de soi que l'invention ne réside pas dans ces différentes formules types. Leur élaboration est à la portée de l'homme de l'art connaissant les produits de coloration d'une gamme de produit et les valeurs des paramètres caractérisant les cheveux à colorer.

Exemple

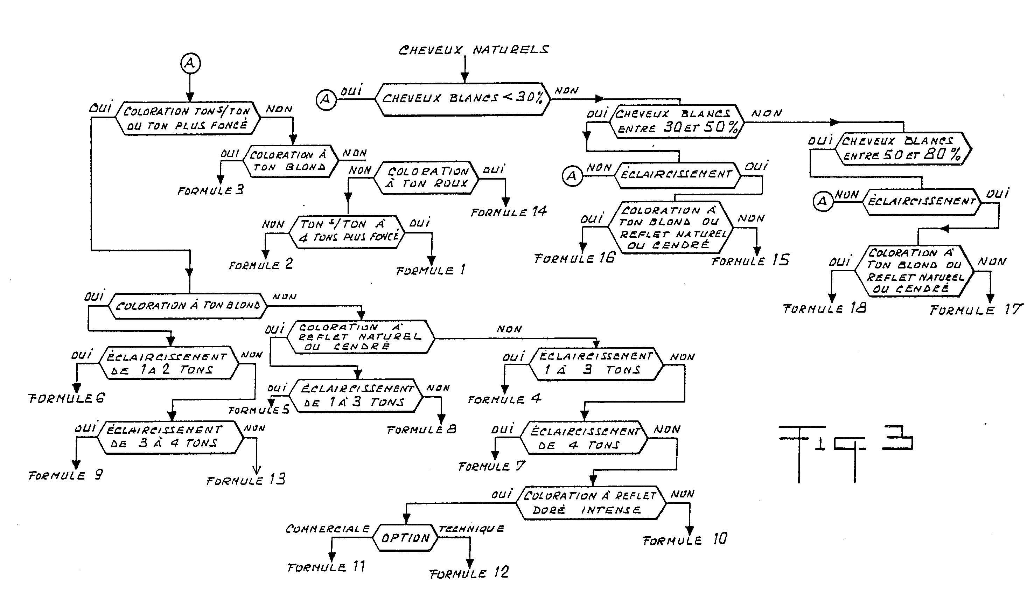

La figure 3 illustre un exemple de sélection de formules.

Le premier paramètre à évaluer est l'état des cheveux. La figure 3 correspond au cas où les cheveux à colorer sont naturels. L'interrogation se déroule dans l'ordre indiqué plus haut jusqu'à saisie des différents paramètres. L'organigramme de la figure 3 comprend les étapes suivantes :

- examen du pourcentage de cheveux blancs,
- pour chaque valeur du paramètre "pourcentage decheveux blancs", calcul de la hauteur de ton à franchir, c'est-à-dire de la différence entre le ton des cheveux à colorer et le ton correspondant à la coloration souhaitée;
- quel que soit le pourcentage de cheveux blancs : si la hauteur de ton calculée va de ton sur ton à 4 tons plus foncé, et si le ton de la coloration désirée n'est pas un blond ou roux, sélection de la formule type 1; si la hauteur de ton calculée est de 5 tons plus foncés et plus, et si le ton de la coloration désirée n'est pas un blond ou roux, sélection de la formule type 2; si la hauteur de ton calculée est ton sur ton ou plus foncé, et si le ton de la coloration désirée est un blond, sélection de la formule type 3;
- pour un pourcentage de cheveux blancs inférieurs à 30 % : si la hauteur de ton correspond à un éclaircissement de 1 à 3 tons, et si la coloration désirée a un ton qui n'est pas un blond et un reflet ni naturel ni cendré, sélection de laformule type 4; si la hauteur de ton correspond à un éclaircissement de 1 à 3 tons et si la coloration demandée a un reflet naturel ou cendré, sélection de la formule type 5 ; si la hauteur de ton correspond à un éclaircissement de 1 à 2 tons et si le ton de la coloration désirée est un blond, sélection de la formule 6; si la hauteur de ton correspond à un éclaircissement de 4 tons et la coloration désirée à un ton qui n'est pas un blond et un reflet ni naturel ni cendré, sélection de la formule 7; si la hauteur de ton correspond à un éclaircissement de 4 tons et plus et si la coloration désirée a un reflet naturel ou cendré, sélection de la formule 8; si la hauteur de ton correspond à un éclaircissement de 3 à 4 tons et si la coloration désirée a un ton blond, sélection de la formule 9; si la hauteur de ton correspond à un éclaircissement de 5 tons et plus et que la coloration désirée n'a pas un ton blond ou un reflet doré avec éclat intense, sélection de la formule 10; si la hauteur de ton correspond à un éclaircissement de 5 tons et plus et que la coloration désirée a un reflet doré avec éclat intense, sélection de la formule 11 ou de la formule 12 selon que l'option choisie est commerciale ou technique ; si la hauteur de ton correspond à un éclaircissement de 5 tons et plus et que La coloration désirée a un ton blond, sélection de la formule 13 ; si la coloration demandée a un ton roux, sélection de la formule 14 ;
- pour un pourcentage de cheveux blancs compris entre 30 et 50 % : si la hauteur de ton correspond à un éclaircissement et si la coloration désirée n'a pas un ton blond ou un reflet naturel ou cendré, sélection de la formule 15 ; si la hauteur de ton correspond à un éclaircissement et si la coloration désirée a un ton blond ou un reflet naturel ou cendré, sélection de la formule 16 ;
- pour un pourcentage de cheveux blancs compris entre 50 et 80 % : si la hauteur de ton correspond à un éclaircissement et si la coloration désirée n'a pas un ton blond ou un reflet naturel ou cendré, sélection de la formule 17 ; si la hauteur de ton correspond à un éclaircissement et si la coloration désirée a un ton blon ou un reflet naturel ou cendré, sélection de la formule 18.

A titre indicatif, des formules de coloration pourront se présenter sous la forme suivante :

Formule 1 :

Un tube de XXXX + "Oxigenta" 20 volumes, dose pour dose ; temps de pose = 30 minutes, XXXX étant la référence du produit correspondant à la coloration souhaitée, la référence d'un produit dans la gamme de coloration étant identique à la référence sous laquelle la coloration désirée correspondante est saisie au moyen du clavier. Il suffit ainsi, dans ce cas, pour l'édition de la formule particulière de coloration de compléter la formule type n° 1 en insérant la référence identifiant la coloration désirée telle que saisie "Oxigenta" est une eau oxygénée commercialisée sous cette dénomination par la Société Schwarzkoff, co-déposante.

Formule 2 :

prépigmentation avec colorant "Igofleur" xxx, 1 tube de XXXX + "Oxigenta" 10 volumes, dose pour dose, temps de pose 30 minutes "Igofleur" désigne une gamme de colorants cationiques de la Société Schwarzkopf. Le choix de la référence xxx est effectué automatiquement en fonction de la différence de hauteur de ton et du reflet de la coloration désirée.

Formule 11 (option commerciale) :

1 tube de "G10" + 90 ml "Oxigenta" 30 volumes, temps de pose = 30 minutes. Le produit "G10" est ici un produit particulier de la Société Schwarzkopf donnant une coloration de ton blond clair à reflet doré.

Formule 12 (option technique) :

décoloration avec crème à blondir + "Oxigenta" 30 volumes à hauteur de ton de la couleur choisie, 1 tube de XXXX + "Oxigenta" 10 volumes, dose pour dose, temps de pose = 30 minutes.

Formule 15 :

1/2 YYYY + 1/4 NXXX + 1/4 crème à blondir + "Oxigenta" 20 volumes, dose pour dose, temps de pose = 30 minutes.

Dans cette formule XXX identifie, comme précédemment, le produit ayant le même ton que celui de la coloration désirée, NXXX signifiant que, parmi ces produits de même ton, on ajoute nécessairement celui ayant un reflet naturel, tandis que YYYY identifie le produit ayant un ton plus foncé que celui de la coloration désirée (la référence de ce produit peut être immédiatement trouvée si, dans le fichier des produits de la gamme mémorisés, à chaque produit on a associé la référence du produit qui s'en distingue simplement par un écart d'un ton plus foncé).

Formule 16 :

décoloration par produit "B blond" + "Oxigenta" 30 volumes 2 tons plus clairs que la couleur choisie ; 1 tube de XXXX + "Oxigenta" 10 volumes, dose pour dose, temps de pose = 30 minutes.

Dans cette formule, "B blond" est un produit super éclaircissement de la société Schwarzkopf.

L'exemple donné ci-dessus correspond à une procédure simplifiée dans la mesure où ni la grosseur des cheveux, ni la qualité des pointes ne sont pris en compte.

Bien entendu, des procédures analogues pourront être élaborées dans le cas de coloration sur cheveux colorés par oxydation, d'éclaircissement sur cheveux colorés sans oxydation, de recoloration de cheveux décolorés, de coloration en ton blond très clair ou en ton blanc ou platine sur cheveux colorés, ...Pour la plupart de ces procédures, il sera tenu compte de la valeur du paramètre "longueur de racines".

Enfin, bien que les exemples donnés font référence à des produits d'une gamme commerciale particulière, il va de soi que l'invention peut être mise en oeuvre avec des produits d'une autre gamme commerciale ou de plusieurs gammes différentes, l'homme de l'art étant à même de déterminer les formules types convenant pour résoudre les différents problèmes posés compte tenu des spécificités des produits de coloration susceptibles d'être employés.

## Revendications

1. Appareil pour déterminer automatiquement la formule de coloration de cheveux afin d'obtenir une coloration souhaitée, caractérisé en ce qu'il comporte :
- une unité de traitement à microprocesseur (10),
- au moins une mémoire (16) contenant des formules de coloration,
- des moyens de saisie de données (20) pour fournir à l'unité de traitement une information représentative de la coloration souhaitée et des informations d'état chacune représentative de la valeur d'un paramètre parmi un ensemble de paramètres prédéterminés, caractérisant les cheveux à colorer, et
- un dispositif d'affichage (30),
- l'unité de traitement comprenant des moyens pour sélectionner une formule de coloration en fonction des données saisies et pour commander l'affichage de la formule sélectionnée sur le dispositif d'affichage.

2. Appareil selon la revendication 1, caractérisé en ce qu'il comprend des moyens d'interrogation pour demander la saisie des informations d'état dans un ordre prédéterminé.

3. Appareil selon l'une quelconque des revendications 1 et 2, caractérisé en ce que les moyens de saisie de données comportent un clavier (20) comprenant des touches dont chacune correspond à une valeur particulière d'un desdits paramètres.

4. Appareil selon la revendication 3, caractérisé en ce que lesdites touches (22) sont groupées en plusieurs ensembles correspondant chacun à un desdits paramètres, et des moyens indicateurs sont associés aux ensembles de touches pour indiquer le paramètre dont la valeur doit être saisie afin de fournir les informations d'état à l'unité de traitement dans un ordre prédéterminé.

5. Appareil selon l'une quelconque des revendications 3 et 4, caractérisé en ce que lesdites touches (22) constituent également des touches d'un clavier alphanumérique.

6. Appareil selon l'une quelconque des revendications 1 à 5, caractérisé en ce que lesdits paramètres sont choisis parmi un ensemble comprenant notamment l'état des cheveux, la hauteur de ton des cheveux, le reflet des longueurs, la longueur des racines, le pourcentage de cheveux blancs, et la grosseur des cheveux.

7. Appareil selon l'une quelconque des revendications 1 à 6, caractérisé en ce que le dispositif d'affichage comprend une imprimante (30) de manière à fournir la formule de coloration sous forme imprimée.

8. Appareil selon l'une quelconque des revendications 1 à 7, caractérisé en ce que l'unité de traitement (10), la mémoire (16), les moyens de saisie de données (20) et le dispositif d'affichage (30) sont regroupés dans un même boitier (50).

9. Appareil selon la revendication 8, caractérisé en ce qu'il comprend en outre une palette (52) montrant les différentes valeurs pouvant être saisies pour la hauteur de ton de cheveux constituant l'un desdits paramètres.

0290327

Fig. 1

FIG. 2

NOM : MME MARCHAND
COULEUR DÉSIRÉE : B6
DIAGNOSTIC CONDENSÉ
: B6 2L RW Y D
- - - - - - - - - - - - -
- - - - - - - - - - - - -
DATE :

NOUVEAU DIAGNOSTIC ►

ETAT CHEVEUX
A
B
C
D

LONGUEUR RACINES
E
F
G

HAUTEUR TON COL.
H
I
J
K
L
M
N
O
P
Q
HAUTEUR TON NATUREL

% CHEVEUX BLANCS
R
S
T
U

GROSSEUR CHEVEUX
V
W
X

QUALITÉ POINTES
Y
Z

REFLET LONGUEURS
0
1
2
3
4
5
6

CAS SPÉCIFIQUE
7
8
9

ORDINATEUR COLORATION

FORMULE
COMMERCIALE
TECHNIQUE

RETOUR ◄
CONFIRME

NOIR | CHATAIN FONCÉ | CHATAIN MOYEN | CHATAIN CLAIR | BLOND FONCÉ | BLOND MOYEN | BLOND CLAIR | BLOND TRÈS CLAIR | PLATINE | ROUX

0290327

Fig. 3

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | PATENT ABSTRACTS OF JAPAN, vol. 2, no. 23 (E-77), 15 février 1978, page 11642 E 77; & JP-A-52 143 729 (TATEISHI DENKI K.K.) 30-11-1977 * Abrégé * --- | 1,2 | G 06 F  15/02 A 45 D  44/00 |
| A | US-A-4 160 271  (GRAYSON et al.) * Colonne 2, lignes 11-48 * --- | 1-4 | |
| A | CH-A-  424 557  (L'OREAL) * Page 1, lignes 34-39 * ----- | | |

| | | | DOMAINES TECHNIQUES RECHERCHES (Int. Cl.4) |
|---|---|---|---|
| | | | G 06 F A 45 D |

Le présent rapport a été établi pour toutes les revendications

| Lieu de la recherche | Date d'achèvement de la recherche | Examinateur |
|---|---|---|
| LA HAYE | 03-08-1988 | SONIUS M.E. |

EPO FORM 1503 03.82 (P0402)